# EUROPEAN PATENT SPECIFICATION

(11) **EP 3 181 164 B1**
(45) Date of publication and mention of the grant of the patent: **15.08.2018**
(21) Application number: 16204182.6
(22) Date of filing: 14.12.2016
(51) Int. Cl.: A61M 1/16, A61M 1/32, A61M 1/34, A61M 1/36, A61M 1/10

(54) **APPARATUS FOR THE DECAPNEIZATION OF BLOOD**
VORRICHTUNG ZUR DECAPNEISIERUNG VON BLUT
APPAREIL D'ÉLIMINATION DU GAZ CARBONIQUE DANS LE SANG

(30) Priority: 18.12.2015 IT UB20159161
(43) Date of publication of application: 21.06.2017
(73) Proprietor: Eurosets S.r.l., 41036 Medolla (MO) (IT)
(72) Inventor: Petralia, Antonio, 48022 Lugo (RA) (IT); Ghelli, Nicola, 40018 S. Pietro in Casale (BO) (IT); Fontanili, Paolo, 42015 Correggio (RE) (IT)
(74) Representative: Zoli, Filippo

(56) References cited:
- WO-A1-2004/089444
- JP-A- 2007 167 672
- YORGIN P D ET AL: "WHERE SHOULD THE HEMOFILTRATION CIRCUIT BE PLACED IN RELATION TO THE EXTRACORPOREAL MEMBRANE OXYGENATION CIRCUIT?", ASAIO JOURNAL, LIPPINCOTT WILLIAMS & WILKINS / ASAIO, HAGERSTOWN, MD, US, vol. 38, no. 4, 1 October 1992 (1992-10-01), pages 801-803, XP000360441, ISSN: 1058-2916

## Description

The present invention relates to an apparatus for the decapneization of blood. As known, decapneization is a substitutive respiratory therapy during which oxygen is supplied to the blood and, at the same time, carbon dioxide in excess is eliminated.

This type of therapy is generally implemented by means of the so-called "oxygenators", which are precisely suitable for removing CO2 from the incoming blood and enriching it with oxygen.

Therapies are also known for the filtering of blood providing for the removal of waste substances that have accumulated in the blood for various reasons such as: pathological causes, surgical causes, administration of substances or other. These therapies are performed by means of so-called "blood-filtering machines", which implement the functions normally carried out by healthy kidneys in correct working conditions.

One therapy of this type is, e.g., CRRT (acronym for Continuous Renal Replacement Therapy).

This kind of machines, to which the patient remains connected also for long periods of time, comprise a filtering device, through which the blood to be treated is conveyed and which is connected to the patient by means of a supply line, adapted to transport the blood to be purified that is drawn from a vein of the patient, and a return line, by means of which the purified blood is reinfused into the patient.

The extra-renal therapy often needs to be associated with the substitutive respiratory therapy.

The relevant machines generally comprise a circuit that is composed of a supply line of the blood to be treated, a device for the oxygenation of blood, a filtering device, adapted to remove the waste substances present in the patient, and of a return line for the reinfusion of the treated blood into the patient.

Some types of apparatus for the decapneization of blood are known from EP 1 415 673 and EP 1 698 362.

EP 1 415 673 describes an apparatus for the decapneization in which the blood to be treated is pushed, by means of a pump, in sequence through the oxygenator and the hemofilter before being reinfused into the patient.

EP 1 698 362 describes, on the other hand, an apparatus for the decapneization in which the oxygenator and the hemofilter are locked together to form a single body piece.

These apparatuses of known type do have some drawbacks.

One drawback consists in the fact that the blood running through the supply line is constrained to pass through both the oxygenator and the hemofilter in sequence. This makes, however, impractical the simultaneous use of the two devices for a long time, because the oxygenator and the hemofilter are characterized by different operating ranges, in particular the oxygenator has a greater operating range (4/5 days approx.) than the hemofilter (24 hours approx.).

This drawback implies using the machine for the decapneization depending on the operating range of the hemofilter or replacing or by-passing the hemofilter while maintaining, at the same time, the oxygenator functioning.

In the cases in which the circuit configuration makes this possible, the hemofilter can be by-passed, which can, however, lead to the formation of blood stasis and clots which can impair the correct operation of the oxygenator. Yorgin et al. (Where should the hemofiltration circuit be placed in relation to the extracorporeal membrane oxygenation circuit?; ASAIO JOURNAL, vol. 38, no. 4; 1992-10-01) discloses a circuit for blood oxygenation with a hemofilter along a bypass line upstream from the oxygenator. The main aim of the present invention is to provide an apparatus for the decapneization of blood which allows controlling the filtration of blood irrespective of the oxygenation thereof.

Within this aim, one object of the present invention is to maintain the oxygenator functioning also during the replacement or maintenance of the hemofilter.

More particularly, one object of the present invention is to avoid, during the operation of the oxygenator and the simultaneous exclusion of the hemofilter, the formation of blood stasis.

Another object of the present invention is to provide an apparatus for the decapneization of blood which allows to overcome the mentioned drawbacks of the prior art within the ambit of a simple, rational, easy, effective to use and affordable solution.

The above mentioned objects are achieved by the present apparatus according to claim 1.

Other characteristics and advantages of the present invention will become better evident from the description of a preferred, but not exclusive, embodiment of an apparatus for the decapneization of blood, illustrated by way of an indicative, but non-limiting, example in the accompanying drawings, wherein:

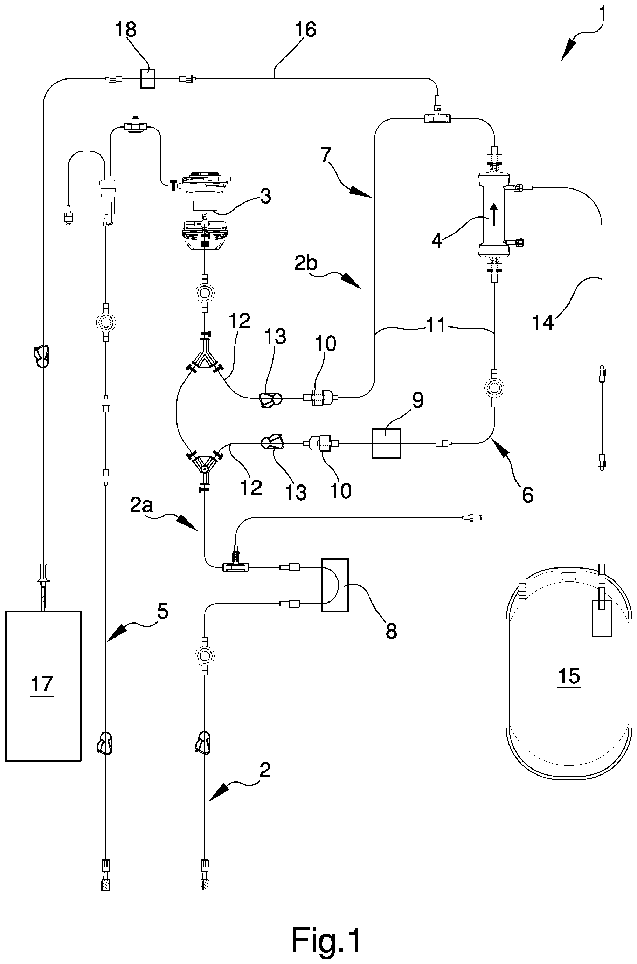

Figure 1 is a schematic view of an apparatus according to the invention.

With particular reference to such illustrations, globally indicated with reference numeral 1 is an apparatus for the decapneization of blood.

The apparatus 1 comprises a supply line 2 of the blood to be treated taken from a patient, at least an oxygenation device 3 for the blood connected at input to the supply line 2, at least a filtering device 4 arranged along the supply line itself and at least a return line 5 connected at output to the oxygenation device 3 and adapted to transport the treated blood to the patient.

The oxygenation device 3 and the filtering device 4 are therefore both arranged along the supply line 2.

The supply line 2 comprises a main section, identified in the figures with reference numeral 2a, connectable on one side to the patient and connected on the opposite side to the input of the oxygenation device 3, and a by-pass section, identified in the figures with reference numeral 2b, arranged parallel to the main section 2a and along which the filtering device 4 is arranged.

The by-pass section 2b therefore has a delivery branch connected on one side to the main section 2a and on the opposite side to the input of the filtering device 4, and a return branch connected on one side to the output of the filtering device 4 and on the opposite side to the main section 2a. The delivery branch and the return branch are identified in the figures with reference numerals 6 and 7, respectively.

Advantageously, the apparatus 1 comprises first pumping means 8 arranged along the main section 2a and adapted to convey the blood to be treated towards the oxygenation device 3.

The first pumping means 8 are arranged upstream of the by-pass section 2b, i.e. upstream of the delivery branch 6, with respect to the direction of advancement of the blood towards the oxygenation device 3.

The first pumping means 8 are of the type of a peristaltic pump or, alternatively, of a centrifugal pump. In the latter case, the centrifugal pump allows a prolonged use of the apparatus 1.

Conveniently, the apparatus 1 also comprises second pumping means 9 arranged along the by-pass section 2b and adapted to convey the blood towards the filtering device 4.

According to the invention, removable connection means 10 are arranged along the delivery branch 6 and along the return branch 7, which are adapted to allow the separation of a first portion 11 of each of the branches 6 and 7 from a relevant second portion 12, where the second portions 12 are connectable to each other by means of the connection means themselves following the separation from the relevant first portions 11.

By joining the second portions 12 together, a section is therefore defined that extends parallel to the main section 2a and which is devoid of blood treatment devices.

Conveniently, the second pumping means 9 are arranged along the first portion 11 of the delivery branch 6, upstream of the filtering device 4 with respect to the direction of blood flow.

Along the by-pass section 2b, and more particularly along the relevant second portions 12, closing means 13 are arranged, of the type of clamps or the like, operable to block the relevant blood passage channels and consequently isolate the filtering device 4. The closing means 13 are therefore arranged along the delivery branch 6 and along the return branch 7, respectively upstream and downstream of the relevant connection means 10.

Following the mutual linkage of the connection means 10, the closing means 13 are again operated to allow the passage of blood through the relevant second portions 12.

The apparatus 1 also comprises a discharge line 14 of the waste substances filtered by the filtering device 4, also called ultrafiltrate, which is connected on one side to the filtering device itself and on the other side to a collection bag 15. Preferably an infusion line 16 is also provided of at least one balancing or restoring substance connected to the by-pass section 2b.

More particularly, the infusion line 16 is connected on one side to a bag 17 containing the balancing substance to infuse and on the opposite side to the by-pass section 2b, at the first portion 11 of the return branch 7, downstream of the filtering device 4.

Conveniently, third pumping means 18 are arranged along the infusion line 16 adapted to convey the balancing substance from the bag 17 towards the by-pass section 2b.

The operation of the present invention is as follows.

In normal operating conditions, i.e. in the case in which the oxygenation device 3 and the filtering device 4 are both functioning, the blood taken from the patient is sent through the first pumping means 8 along the main section 2a towards the oxygenation device 3.

More in detail, one part of the blood that runs through the main section 2a reaches the oxygenation device 3 and one part is deflected, through the second pumping means 9, along the by-pass section 2b. The part of blood that runs through the delivery branch 6 of the by-pass section 2b is therefore sent by the second pumping means 9 towards the filtering device 4, later on going back inside the main section 2a through the return branch 7.

When it is necessary to replace the filtering device 4, in order to maintain the oxygenation device 3 functioning, the by-pass section 2b is blocked by means of the closing means 13.

Subsequently the first portions 11 together with the filtering device 4 are removed from the connection means 10 and the second portions 12 are connected to one another. The second portions 12 connected in this manner then define a section parallel to the main section 2a and devoid of blood treatment devices. Once the passage channel defined by the second portions 12 is reopened, again through the closing means 13, the blood taken from the patient is then entirely sent to the oxygenation device 3 by means of the first pumping means 8.

When the operator wishes to restore the complete functionality of the apparatus 1, by inserting again the filtering device 4, he/she should perform the aforementioned operations but in the reverse sequence. More in detail, the second portions 12 are blocked by means of the closing means 13, so as to prevent the passage of blood through the by-pass section 2b, and the second portions are separated from each other by means of the connection means 10. Subsequently the first portions 11, along which a new filtering device 4 and the second pumping means 9 are positioned, are connected again to the relevant second portions 12 by means of the connection means 10.

It has in practice been ascertained that the described invention achieves the intended objects and in particular the fact is underlined that the presence of a by-pass section arranged parallel to the main section and connecting the patient to the oxygenation device allows using the filtering device as an optional treatment, without therefore constraining the filtering step to the oxygenation one.

More in particular, the presence of the second pumping means arranged along the by-pass section allows controlling the blood flow rate which undergoes the filtering treatment according to the specific requirements.

A further advantage of the apparatus to which the present invention relates consists in the fact that the presence of the connection means arranged along the by-pass section allows separating the latter from the main section, in order to allow the replacement of the filtering device, at the same time maintaining the oxygenation device functioning.

## Claims

1. Apparatus (1) for the decapneization of blood, comprising:
- a supply line (2) of the blood to be treated taken from a patient and at least a return line (5) adapted to transport the treated blood to the patient;
- at least an oxygenation device (3) and at least a filtering device (4) of the blood positioned between said supply line (2) and said return line (5);
where said supply line (2) comprises a main section (2a), connectable on one side to the patient and connected on the opposite side to said oxygenation device (3), and a by-pass section (2b), arranged parallel to said main section (2a) and along which said filtering device (4) is arranged,
**characterized by** the fact that said by-pass section (2b) comprises a delivery branch (6), connected on one side to said main section (2a) and on the other side to the input of said filtering device (4), and a return branch (7), connected on one side to the output of the filtering device (4) and on the other side to said main section (2a), and by the fact that it comprises removable connection means (10), arranged along said branches (6, 7) and adapted to allow the separation of a first portion (11) of the branches themselves which is connected to said filtering device (4), from a respective second portion (12) connected to said main section (2a), said second portions (12) being connectable to each other by means of the connection means themselves following the separation from their respective first portions (11).

2. Apparatus (1) according to claim 1, **characterized by** the fact that it comprises first pumping means (8) arranged along said main section (2a), upstream of said by-pass section (2b), and adapted to convey the blood to be treated towards said oxygenation device (3).

3. Apparatus (1) according to claim 2, **characterized by** the fact that said first pumping means (8) are of the type of a centrifugal pump.

4. Apparatus (1) according to one or more of the preceding claims, **characterized by** the fact that it comprises second pumping means (9) arranged along said by-pass section (2b) and adapted to convey the blood to be treated towards said filtering device (4).

5. Apparatus (1) according to one or more of the preceding claims, **characterized by** the fact that said second pumping means (9) are arranged downstream of said connection means (10) along the first portion (11) of said delivery branch and upstream of the filtering device (4).

6. Apparatus (1) according to one or more of the preceding claims, **characterized by** the fact that it comprises closing means (13) of said by-pass section (2b) arranged along said second portions (12).

7. Apparatus (1) according to one or more of the preceding claims, **characterized by** the fact that it comprises at least a discharge line (14) of the waste substances filtered by said filtering device (4), connected on one side to the filtering device itself and connectable on the other side to a collection bag (15).

8. Apparatus (1) according to one or more of the preceding claims, **characterized by** the fact that it comprises at least one infusion line (16) of at least one balancing substance connected to said by-pass section (2b).

9. Apparatus (1) according to claim 8, **characterized by** the fact that said infusion line (16) is connected to said by-pass section (2b) at the first portion (11) of said return branch (7), downstream of said filtering device (4).

10. Apparatus (1) according to claim 8 or 9, **characterized by** the fact that it comprises third pumping means (18) arranged along said infusion line (16).

## Patentansprüche

1. Vorrichtung (1) zur Decapneisierung von Blut, umfassend:
- eine Zufuhrleitung (2) des zu behandelnden Bluts, das einem Patienten entnommen wurde, und mindestens eine Rückführleitung (5), die geeignet ist, das behandelte Blut zum Patienten zu transportieren;
- mindestens eine Sauerstoffanreicherungsvorrichtung (3) und mindestens eine Filtervorrichtung (4) des Bluts, die zwischen der Zufuhrleitung (2) und der Rückführleitung (5) positioniert ist,
wobei die Zufuhrleitung (2) eine Hauptsektion (2a), die auf einer Seite mit dem Patienten verbunden werden kann und auf der gegenüberliegenden Seite mit der Sauerstoffanreicherungsvorrichtung (3) verbunden ist, und eine Bypass-Sektion (2b), die parallel zur Hauptsektion (2a) angeordnet ist, und entlang derer die Filtervorrichtung (4) angeordnet ist, umfasst,
**dadurch gekennzeichnet, dass** die Bypass-Sektion (2b) umfasst: eine Abgabeverzweigung (6), die auf einer Seite mit der Hauptsektion (2a) und auf der anderen Seite mit dem Eingang der Filtervorrichtung (4) verbunden ist, und eine Rückführverzweigung (7), die auf einer Seite mit dem Ausgang der Filtervorrichtung (4) und auf der anderen Seite mit der Hauptsektion (2a) verbunden ist, und dadurch, dass diese entfernbare Verbindungsmittel (10) umfasst, die entlang der Verzweigungen (6, 7) angeordnet und geeignet sind, die Trennung eines ersten Abschnitts (11) der Verzweigungen selbst, der mit der Filtervorrichtung (4) verbunden ist, von einem jeweiligen zweiten Abschnitt (12), der mit der Hauptsektion (2a) verbunden ist, zu gestatten, wobei die zweiten Abschnitte (12) durch die Verbindungsmittel selbst nach der Trennung von ihren jeweiligen ersten Abschnitten (11) miteinander verbunden werden können.

2. Vorrichtung (1) nach Anspruch 1,
**dadurch gekennzeichnet, dass** diese erste Pumpenmittel (8) umfasst, die entlang der Hauptsektion (2a), stromaufwärts von der Bypass-Sektion (2b), angeordnet und geeignet sind, das zu behandelnde Blut zur Sauerstoffanreicherungsvorrichtung (3) zu befördern.

3. Vorrichtung (1) nach Anspruch 2,
**dadurch gekennzeichnet, dass** die ersten Pumpenmittel (8) vom Typ einer Zentrifugalpumpe sind.

4. Vorrichtung (1) nach einem oder mehreren der vorhergehenden Ansprüche,
**dadurch gekennzeichnet, dass** diese zweite Pumpenmittel (9) umfasst, die entlang der Bypass-Sektion (2b) angeordnet und geeignet sind, das zu behandelnde Blut zur Filtervorrichtung (4) zu befördern.

5. Vorrichtung (1) nach einem oder mehreren der vorhergehenden Ansprüche,
**dadurch gekennzeichnet, dass** die zweiten Pumpenmittel (9) stromabwärts von den Verbindungsmitteln (10) entlang des ersten Abschnitts (11) der Abgabeverzweigung und stromaufwärts von der Filtervorrichtung (4) angeordnet sind.

6. Vorrichtung (1) nach einem oder mehreren der vorhergehenden Ansprüche,
**dadurch gekennzeichnet, dass** diese Verschlussmittel (13) der Bypass-Sektion (2b) umfasst, die entlang der zweiten Abschnitte (12) angeordnet sind.

7. Vorrichtung (1) nach einem oder mehreren der vorhergehenden Ansprüche,
**dadurch gekennzeichnet, dass** diese mindestens eine Ablassleitung (14) der Abfallsubstanzen umfasst, die von der Filtervorrichtung (4) gefiltert werden, welche auf einer Seite mit der Filtervorrichtung selbst verbunden ist und auf der anderen Seite mit einem Sammelbeutel (15) verbunden werden kann.

8. Vorrichtung (1) nach einem oder mehreren der vorhergehenden Ansprüche,
**dadurch gekennzeichnet, dass** diese mindestens eine Infusionsleitung (16) mindestens einer Ausgleichssubstanz umfasst, die mit der Bypass-Sektion (2b) verbunden ist.

9. Vorrichtung (1) nach Anspruch 8,
**dadurch gekennzeichnet, dass** die Infusionsleitung (16) mit der Bypass-Sektion (2b) am ersten Abschnitt (11) der Rückführverzweigung (7), stromabwärts von der Filtervorrichtung (4), verbunden ist.

10. Vorrichtung (1) nach Anspruch 8 oder 9,
**dadurch gekennzeichnet, dass** dieses dritte Pumpenmittel (18) umfasst, die entlang der Infusionsleitung (16) angeordnet sind.

## Revendications

1. Appareil (1) pour l'élimination du gaz carbonique dans le sang, comprenant :
- une ligne d'alimentation (2) du sang à traiter prélevé d'un patient et au moins une ligne de retour (5) adaptée pour transporter le sang traité au patient;
- au moins un dispositif d'oxygénation (3) et au moins un dispositif de filtrage (4) du sang positionnés entre ladite ligne d'alimentation (2) et ladite ligne de retour (5) ;
où ladite ligne d'alimentation (2) comprend une section principale (2a), pouvant être reliée sur un côté au patient et reliée sur le côté opposé audit dispositif d'oxygénation (3), et une section de dérivation (2b), agencée parallèlement à ladite section principale (2a) et le long de laquelle ledit dispositif de filtrage (4) est agencé, **caractérisé par le fait que** ladite section de dérivation (2b) comprend une branche de distribution (6), reliée sur un côté à ladite section principale (2a) et sur l'autre côté à l'entrée dudit dispositif de filtrage (4), et une branche de retour (7), reliée sur un côté à la sortie du dispositif de filtrage (4) et sur l'autre côté à ladite section principale (2a), et **par le fait qu'**il comprend un moyen de liaison amovible (10), agencé le long desdites branches (6, 7) et adapté pour permettre la séparation d'une première portion (11) des branches elles-mêmes qui est reliée audit dispositif de filtrage (4), d'une deuxième portion respective (12) reliée à ladite section principale (2a), lesdites deuxièmes portions (12) pouvant être reliées entre elles à l'aide du moyen de liaison lui-même à la suite de la séparation vis-à-vis de leurs premières portions respectives (11).

2. Appareil (1) selon la revendication 1, **caractérisé par le fait qu'**il comprend un premier moyen de pompage (8) agencé le long de ladite section principale (2a), en amont de ladite section de dérivation (2b), et adapté pour acheminer le sang à traiter vers ledit dispositif d'oxygénation (3).

3. Appareil (1) selon la revendication 2, **caractérisé par le fait que** ledit premier moyen de pompage (8) est du type d'une pompe centrifuge.

4. Appareil (1) selon une ou plusieurs des revendications précédentes, **caractérisé par le fait qu'**il comprend un deuxième moyen de pompage (9) agencé le long de ladite section de dérivation (2b) et adapté pour acheminer le sang à traiter vers ledit dispositif de filtrage (4).

5. Appareil (1) selon une ou plusieurs des revendications précédentes, **caractérisé par le fait que** ledit deuxième moyen de pompage (9) est agencé en aval dudit moyen de liaison (10) le long de la première portion (11) de ladite branche de distribution et en amont du dispositif de filtrage (4).

6. Appareil (1) selon une ou plusieurs des revendications précédentes, **caractérisé par le fait qu'**il comprend un moyen de fermeture (13) de ladite section de dérivation (2b) agencée le long desdites deuxièmes portions (12).

7. Appareil (1) selon une ou plusieurs des revendications précédentes, **caractérisé par le fait qu'**il comprend au moins une ligne d'évacuation (14) de substances de déchets filtrées par ledit dispositif de filtrage (4), reliée sur un côté au dispositif de filtrage lui-même et pouvant être reliée sur l'autre côté à une poche de collecte (15).

8. Appareil (1) selon une ou plusieurs des revendications précédentes, **caractérisé par le fait qu'**il comprend au moins une ligne de perfusion (16) d'au moins une substance d'équilibrage reliée à ladite section de dérivation (2b).

9. Appareil (1) selon la revendication 8, **caractérisé par le fait que** ladite ligne de perfusion (16) est reliée à ladite section de dérivation (2b) au niveau de la première portion (11) de ladite branche de retour (7), en aval dudit dispositif de filtrage (4).

10. Appareil (1) selon la revendication 8 ou 9, **caractérisé par le fait qu'**il comprend un troisième moyen de pompage (18) agencé le long de ladite ligne de perfusion (16).
